# EUROPEAN PATENT APPLICATION

(11) **EP 4 732 809 A1**
(43) Date of publication of application: **29.04.2026**
(21) Application number: 25210599.4
(22) Date of filing: 23.10.2025
(51) Int. Cl.: A61F 2/24

(54) **HEART VALVE PROSTHESES AND TRANSCATHETER DEVICES**

(30) Priority: 23.10.2024 US 202463710936 P; 18.08.2025 US 202519302272
(71) Applicant: Medtronic, Inc., Minneapolis, Minnesota 55432 (US)
(72) Inventor: Griswold, Erik C., Minneapolis, 55432 (US)
(74) Representative: Zimmermann & Partner Patentanwälte mbB

(57) **Abstract**

A heart valve prosthesis that includes an expandable anchoring frame having an interior area extending between a proximal portion of the expandable anchoring frame and a distal portion of the expandable anchoring frame. An expandable stent frame includes an interior area. A prosthetic valve structure is mounted within the interior area of the expandable stent frame. The expandable stent frame is configured to be inserted into the interior area of the expandable anchoring frame and, when the expandable stent frame is in an expanded configuration, the expandable stent frame is biased radially outward toward an inner surface of the expandable anchoring frame and is dimensioned to capture the at least one native leaflet between an outer surface of the expandable stent frame and the inner surface of the expandable anchoring frame.

## Description

### CROSS-REFERENCE TO RELATED APPLICATIONS

This application claims the benefit of U.S. Provisional Patent Application Serial No. 63/710,936, filed October 23, 2024, and U.S. Patent Application Serial No. 19/302,272, filed August 18, 2025 the entire contents of which are incorporated herein by reference.

### FIELD

The present disclosure relates generally to heart valve prostheses, transcatheter devices and methods, and more particularly, to transcatheter devices and methods of implanting a stented prosthesis.

### BACKGROUND

A human heart includes four valves that determine the pathway of blood flow through the heart: the mitral valve, the tricuspid valve, the aortic valve, and the pulmonary valve. The mitral and tricuspid valves are atrio-ventricular valves, which connect the atria to the ventricles, while the aortic and pulmonary valves are semilunar valves located between the ventricles and their corresponding artery and regulate the flow of blood leaving the heart. Each of the valves are made from thin, strong flaps of tissue called leaflets. Ideally, native leaflets of a heart valve move apart from each other when the valve is an open position and meet or "coapt" when the valve is in a closed position.

Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

Diseased or otherwise deficient heart valves can be repaired or replaced using a variety of different types of heart valve surgeries. One conventional technique involves an open-heart surgical approach that is conducted under general anesthesia, during which the heart is stopped, and blood flow is controlled by a heart-lung bypass machine.

More recently, minimally invasive approaches have been developed to facilitate catheter-based implantation of a prosthetic heart valve or prosthesis on the beating heart, intending to obviate the need for the use of classical sternotomy and cardiopulmonary bypass. In general terms, an expandable prosthetic valve is compressed about or within a catheter, inserted inside a body lumen of the patient, such as a femoral artery, and delivered to a desired location in the heart.

However, the minimally invasive approaches are sometimes less effective for patients who have uncommonly large leaflets. When the prosthetic heart valve is deployed, these leaflets can cause coronary obstruction where the leaflets block the replacement valve when it opens and prevents the flow of blood to the coronary arteries.

In light of the above, a need exists for a prosthetic heart valve and transcatheter device that can manage the leaflets of a heart valve during catheter-based implantations of prosthetic heart valves.

### SUMMARY

The following presents a simplified summary of the disclosure to provide a basic understanding of some aspects described in the detailed description.

In an example, a heart valve prosthesis for capturing at least one native leaflet of a heart valve. The heart valve prosthesis comprises an expandable anchoring frame comprising an interior area extending between a proximal portion of the expandable anchoring frame and a distal portion of the expandable anchoring frame. An expandable stent frame comprises an interior area extending between a proximal portion of the expandable stent frame and a distal portion of the expandable stent frame. The expandable stent frame comprises a prosthetic valve structure mounted within the interior area of the expandable stent frame. The expandable stent frame is configured to be inserted into the interior area of the expandable anchoring frame and, when the expandable stent frame is in an expanded configuration, the expandable stent frame is biased radially outward toward an inner surface of the expandable anchoring frame and is dimensioned to capture the at least one native leaflet between an outer surface of the expandable stent frame and the inner surface of the expandable anchoring frame.

In an example, a method of implanting the heart valve prosthesis comprising: delivering the heart valve prosthesis to the heart valve of a patient while both the expandable anchoring frame and the expandable stent frame are in contracted orientations; fully deploying the expandable anchoring frame such that at least one leaflet of the heart valve is adjacent the inner surface of the expandable anchoring frame; expanding the expandable anchoring frame; fully deploying the expandable stent frame into the interior area of the expandable anchoring frame such that the at least one leaflet of the heart valve is positioned between the inner surface of the expandable anchoring frame and the outer surface of the expandable stent frame; and expanding the expandable stent frame to capture the at least one leaflet of the heart valve between the expandable anchoring frame and the expandable stent frame.

In an example, a two-component prosthetic valve delivery assembly comprising: a transcatheter delivery assembly comprising: an outer sheath, and an inner member, wherein the outer sheath and the inner member are movable relative to each other in an axial direction. An expandable anchoring frame is constrained by the outer sheath. An expandable stent frame comprises a prosthetic valve structure mounted within an interior area of the expandable stent frame. The expandable stent frame is disposed between the outer sheath and the inner member and is moveable with the inner member. The expandable anchoring frame is configured to be released from the transcatheter delivery assembly prior to the expandable stent frame being released from the transcatheter delivery assembly.

Additional features and advantages of the aspects disclosed herein will be set forth in the detailed description that follows, and in part will be clear to those skilled in the art from that description or recognized by practicing the aspects described herein, including the detailed description which follows, the claims, as well as the appended drawings. It is to be understood that both the foregoing general description and the following detailed description present aspects intended to provide an overview or framework for understanding the nature and character of the aspects disclosed herein. The accompanying drawings are included to provide further understanding and are incorporated into and constitute a part of this specification. The drawings illustrate various aspects of the disclosure, and together with the description explain the principles and operations thereof.

### BRIEF DESCRIPTION OF THE DRAWINGS

These and other features, aspects and advantages are better understood when the following detailed description is read with reference to the accompanying drawings, in which:
FIG. 1 illustrates a side view of an exemplary expandable stent frame of a heart valve prosthesis, in accordance with aspects of the disclosure;
FIG. 2 illustrates a top view of the exemplary expandable stent frame of FIG. 1 taken from an outflow end thereof;
FIG. 3 illustrates a side view of an exemplary expandable anchoring frame of a heart valve prosthesis;
FIG. 4 illustrates a side view of a transcatheter delivery assembly for delivering a heart valve prosthesis, in accordance with aspects of the disclosure;
FIG. 5 illustrates an enlarged side view of a distal portion of the transcatheter delivery assembly of FIG. 4, in accordance with aspects of the disclosure;
FIG. 6 illustrates a section view showing both the expandable stent frame of FIG. 1 and the expandable anchoring frame of FIG. 3 in a contracted condition and positioned within the transcatheter delivery assembly of FIG. 4, according to an aspect of the disclosure;
FIG. 7 illustrates a section view showing both the expandable stent frame of FIG. 1 and the expandable anchoring frame of FIG. 3 in a contracted condition and positioned within the transcatheter delivery assembly of FIG. 4 with the expandable stent frame partially positioned within the expandable anchoring frame, according to an aspect of the disclosure;
FIG. 8 illustrates a section view showing both the expandable stent frame of FIG. 1 and the expandable anchoring frame of FIG. 3 in a contracted condition and positioned within the transcatheter delivery assembly of FIG. 4 with the expandable stent frame fully positioned within the expandable anchoring frame, according to an aspect of the disclosure;
FIG. 9 schematically illustrates distally advancing the transcatheter delivery assembly of FIG. 4 through a heart valve of a patient, according to an aspect of the disclosure;
FIG. 10 schematically illustrates positioning the expandable stent of FIG. 1 (in a contracted condition) in a heart valve of a patient and partially deploying the expandable anchoring frame of FIG. 3, according to an aspect of the disclosure;
FIG. 11 schematically illustrates partially deploying the expandable anchoring frame of FIG. 3 proximate a heart valve of a patient, according to an aspect of the disclosure;
FIG. 12 schematically illustrates fully deploying the expandable anchoring frame of FIG. 3 proximate a heart valve of a patient, according to an aspect of the disclosure;
FIG. 13 schematically illustrates expanding the expandable stent frame of FIG. 1 to capture native leaflets of a heart valve between the expandable stent frame and the expandable anchoring frame of FIG. 3, according to an aspect of the disclosure;
FIG. 14 schematically illustrates withdrawing the transcatheter delivery assembly from a heart of a patient, according to an aspect of the disclosure;
FIG. 15 schematically illustrates distally advancing a transcatheter delivery assembly according to another embodiment through a heart valve of a patient and partially deploying the expandable anchor frame of FIG. 3 from an outer sleeve of the transcatheter delivery assembly, according to an aspect of the disclosure;
FIG. 16 schematically illustrates fully deploying the expandable anchoring frame of FIG. 3 from the outer sleeve of the transcatheter delivery assembly of FIG. 15, according to an aspect of the disclosure;
FIG. 17 schematically illustrates partially deploying the expandable stent frame of FIG. 1 from an inner sleeve of the transcatheter delivery assembly of FIG. 15, according to an aspect of the disclosure;
FIG. 18 schematically illustrates expanding the expandable stent frame of FIG. 1 to capture native leaflets of a heart valve between the expandable stent frame and the expandable anchoring frame of FIG. 3, according to an aspect of the disclosure; and
FIG. 19 schematically illustrates withdrawing the transcatheter delivery assembly of FIG. 15 from a heart of a patient, according to an aspect of the disclosure.

### DETAILED DESCRIPTION

Aspects will now be described more fully hereinafter with reference to the accompanying drawings in which example aspects are shown. Whenever possible, the same reference numerals are used throughout the drawings to refer to the same or like parts. However, this disclosure may be embodied in many different forms and should not be construed as limited to the aspects set forth herein.

As used herein, the term "about" means that amounts, sizes, formulations, parameters, and other quantities and characteristics are not, and need not be, exact, but may be approximate and/or larger or smaller, as desired, reflecting tolerances, conversion factors, rounding off, measurement error and the like, and other factors known to those of skill in the art.

Ranges can be expressed herein as from "about" one value, and/or to "about" another value. When such a range is expressed, aspects include from the one value to the other value. Similarly, when values are expressed as approximations by use of the antecedent "about," it will be understood that the value forms another aspect. It will be further understood that the endpoints of each of the ranges are significant both in relation to the other endpoint, and independently of the other endpoint.

Directional terms as used herein - for example up, down, right, left, front, back, top, bottom, upper, lower, etc. - are made only with reference to the figures as drawn and are not intended to imply absolute orientation.

Unless otherwise expressly stated, it is in no way intended that any methods set forth herein be construed as requiring that its steps be performed in a specific order, nor that with any apparatus, specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred in any respect. This holds for any possible non-express basis for interpretation, including matters of logic relative to arrangement of steps, operational flow, order of components, or orientation of components; plain meaning derived from grammatical organization or punctuation, and; the number or type of aspects described in the specification.

As used herein, the singular forms "a," "an," and "the" include plural references unless the context clearly dictates otherwise. Thus, for example, reference to "a" component includes aspects having two or more such components, unless the context clearly indicates otherwise.

The word "exemplary," "example," or various forms thereof are used herein to mean serving as an example, instance, or illustration. Any aspect or design described herein as "exemplary" or as an "example" should not be construed as preferred or advantageous over other aspects or designs. Furthermore, examples are provided solely for purposes of clarity and understanding and are not meant to limit or restrict the disclosed subject matter or relevant portions of this disclosure in any manner. It can be appreciated that a myriad of additional or alternate examples of varying scope could have been presented but have been omitted for purposes of brevity.

As used herein, the terms "comprising," "including," and variations thereof shall be construed as synonymous and open-ended, unless otherwise indicated. A list of elements following the transitional phrases comprising or including is a non-exclusive list, such that elements in addition to those specifically recited in the list may also be present.

The terms "substantial," "substantially," and variations thereof as used herein are intended to represent that a described feature is equal or approximately equal to a value or description. For example, a "substantially planar" surface is intended to denote a surface that is planar or approximately planar. Moreover, "substantially" is intended to denote that two values are equal or approximately equal. The term "substantially" may denote values within about 10% of each other, for example, within about 5% of each other, or within about 2% of each other.

Modifications may be made to the instant disclosure without departing from the scope or spirit of the claimed subject matter. Unless specified otherwise, "first," "second," or the like are not intended to imply a temporal aspect, a spatial aspect, an ordering, etc. Rather, such terms are merely used as identifiers, names, etc. for features, elements, items, etc. For example, a first end and a second end generally correspond to end A and end B or two different ends.

Unless otherwise indicated, the terms "distal"and "proximal" are used in the following description with respect to a position or direction relative to the treating clinician. "Distal" and "distally" are positions distant from or in a direction away from the clinician, and "proximal" and "proximally" are positions near or in a direction toward the clinician. In addition, the term "self-expanding" may be used in the following description with reference to one or more valve or stent structures of the prostheses hereof and is intended to convey that the structures are shaped or formed from a material that can be provided with a mechanical memory to return the structure from a compressed or constricted delivery configuration to an expanded deployed configuration or vice versa. Non-exhaustive exemplary self-expanding materials include stainless steel, a pseudoelastic metal such as a nickel titanium alloy or nitinol, various polymers, or a so-called super alloy, which may have a base metal of nickel, cobalt, chromium, or other metal. Mechanical memory may be imparted to a wire or stent structure by thermal treatment to achieve a spring temper in stainless steel, for example, or to set a shape memory in a susceptible metal alloy, such as nitinol. Various polymers that can be made to have shape memory characteristics may also be suitable for use in aspects hereof to include polymers such as polynorborene, trans-polyisoprene, styrenebutadiene, and polyurethane. As well poly L-D lactic copolymer, oligo caprylactone copolymer and poly cyclo-octine can be used separately or in conjunction with other shape memory polymers.

Diseases associated with heart valves, such as those caused by damage or a defect, can include stenosis and valvular insufficiency or regurgitation. For example, valvular stenosis causes the valve to become narrowed and hardened which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots which can increase the likelihood of stroke or pulmonary embolism. Symptoms can often be severe enough to be debilitating and/or life threatening.

Heart valve prostheses have been developed for repair and replacement of diseased and/or damaged heart valves. Such heart valve prostheses can be percutaneously delivered and deployed at the site of the diseased heart valve through catheter-based delivery systems. Such heart valve prostheses generally include a frame or stent and a prosthetic valve mounted within the frame. Such heart valve prostheses are delivered in a radially compressed or crimped configuration so that the heart valve prosthesis can be advanced through the patient's vasculature. Once positioned at the treatment site, the heart valve prosthesis is expanded to engage tissue at the diseased heart valve region to, for instance, hold the heart valve prosthesis in position.

In some embodiments, the heart valve prosthesis may be delivered to and implanted at a treatment site within a patient to replace any of an aortic valve, a pulmonic valve, a mitral valve, and a tricuspid valve. The valve to be replaced may be a native valve or a previously-implanted prosthetic valve, such as a failed surgical replacement valve or a failed transcatheter valve. In the present disclosure, the invention will be described with reference to replacing the aortic valve, but as noted above, may similarly apply to replacing the pulmonic valve, the mitral valve or the tricuspid valve.

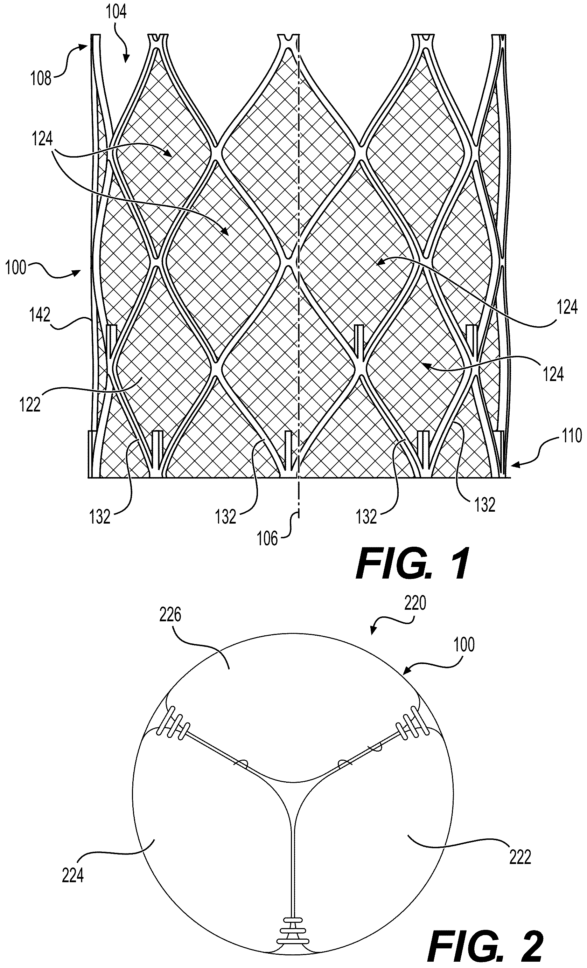

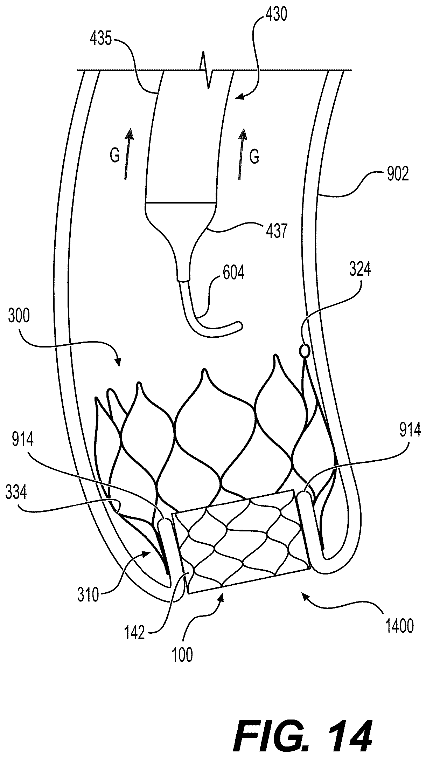

With reference to FIG. 1, in some aspects, an expandable stent frame 100 comprises one component of a transcatheter heart valve prosthesis 1400 (FIG. 14). The expandable stent frame 100 supports a heart valve prosthesis 220 (FIG. 2). The heart valve prosthesis 220 can comprise one or more leaflets (e.g., two or more leaflets). In some examples, as shown, the one or more leaflets can comprise three leaflets to emulate a tricuspid valve. For example, referring to FIG. 2, the one or more leaflets can comprise a first leaflet 222, a second leaflet 224, and a third leaflet 226. In some aspects, the one or more leaflets can comprise two leaflets, such as for example, to emulate a bicuspid valve. Any other suitable number of leaflets may be utilized. The heart valve prosthesis 220 is not required to have the same number of valve structures and/ or leaflets of the native valve structure. The leaflets may be attached to a skirt 122 which encloses or lines a portion of the expandable stent frame 100.

The leaflets of the heart valve prosthesis 220 may be made of pericardial material; however, the leaflets may be fabricated by other materials in further embodiments. For example, leaflets of the disclosure may be fabricated from natural tissue that can be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as the leaflets include DACRON polyester commercially available from Invista North America S.A.R.L. of Wilmington, DE, other cloth materials, nylon blends, polymeric materials, and vacuum deposition nitinol fabricated materials. With certain leaflet materials, it may be desirable to coat one or both sides of the leaflet with a material that will prevent or minimize overgrowth. It is further desirable that the leaflet material is durable and not subject to stretching, deforming, or fatigue.

The skirt 122 may enclose or line the expandable stent frame 100 as would be known to one of ordinary skill in the art of prosthetic tissue valve construction. The skirt 122 may be a natural or biological material such as pericardium or another membranous tissues such as intestinal submucosa. Alternatively, the skirt 122 may be a low-porosity woven fabric, such as polyester, Dacron fabric, or PTFE (polytetrafluoroethylene), which creates a one-way fluid passage when attached to the stent. In one embodiment, the skirt 122 may be a knit or woven polyester, such as a polyester or PTFE knit, which can be utilized when it is desired to provide a medium for tissue ingrowth one side and a smooth surface on the other side.

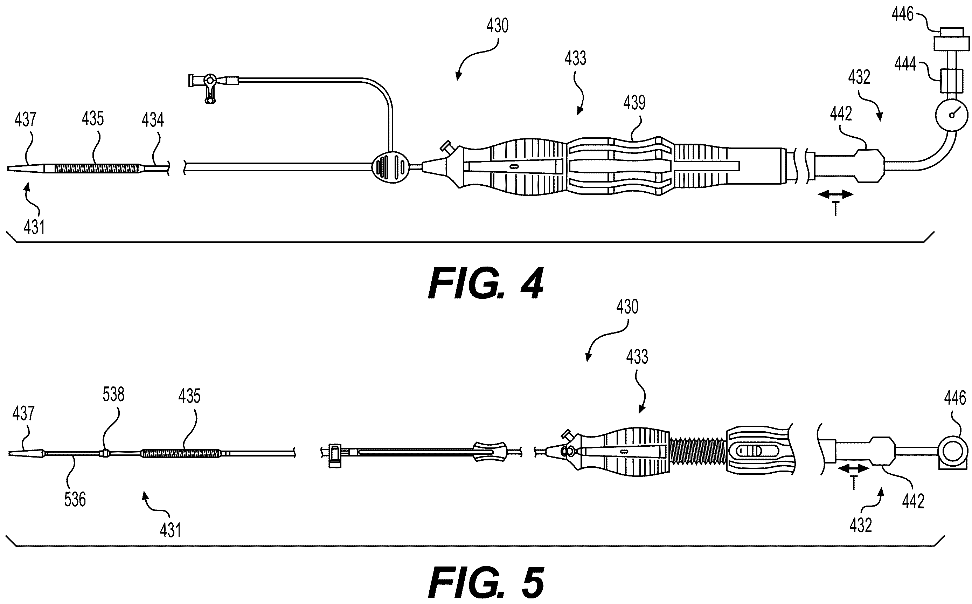

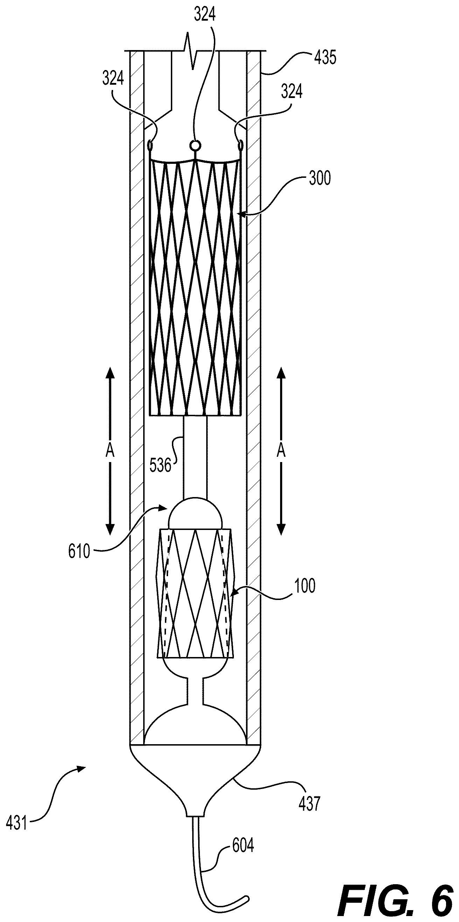

As described in detail below, the expandable stent frame 100 may be a self-expandable stent frame or ballon-expandable. In aspects, the expandable stent frame 100 can comprise an inner lumen 104 extending along an elongated axis 106. In some examples where the expandable stent frame 100 includes the expansion device, the expansion device can be positioned within the inner lumen 104. For example, the expansion device can comprise a balloon configured to receive saline, a contrast solution, or compressed air that may be delivered to the balloon to expand the expandable stent frame 100. In aspects, the expandable stent frame 100 can comprise a proximal end 108 (outflow end) and a distal end 110 (inflow end). The expandable stent frame 100 can extend from the proximal end 108 to the distal end 110. In aspects, the expandable stent frame 100 can be configured to be received within a transcatheter delivery assembly 430 (see, FIG. 4), as described in detail below. In some embodiments, the expandable stent frame 100 can be configured to achieve a contracted orientation (shown in FIG. 6) and a fully expanded orientation (shown in FIG. 1).

The term "fully expanded orientation" should be construed to mean that the expandable stent frame 100 cannot expand any further with respect to the location at which the expandable stent frame 100 has been deployed (i.e., fully released from the transcatheter delivery assembly 430). In some cases, the term "fully expanded orientation" may refer to the expandable stent frame 100 not being capable of any further radial expansion regardless of the location at which the expandable stent frame 100 is deployed (e.g., the expandable stent frame 100 cannot radially expand any further, even when free from surrounding structure).

The heart valve prosthesis 220 is attached to the expandable stent frame 100 such that when pressure at the distal end 110 exceeds pressure at the proximal end 108, the leaflets of the heart valve prosthesis 220 open to allow blood flow through the heart valve prosthesis 220 from the distal end 110 to the proximal end 108. When the pressure at the proximal end 108 exceeds pressure at the distal end 110, the leaflets of the heart valve prosthesis 220 close to prevent blood from the proximal end 108 to the distal end 110.

The expandable stent frame 100 includes a plurality of struts 132 that are arranged to form a plurality of cells 124. The struts 132 are defined herein as the elongated wire segments of the expandable stent frame 100. The expandable stent frame 100, and, thus, the plurality of struts 132, can be adjusted between a radially-collapsed position and a radially-expanded position. In some embodiments, the expandable stent frame 100 is self-expanding and can further comprise nitinol. In the example embodiment shown in FIG. 1, the plurality of cells 124 may be in a shape of a rhombus. The expandable stent frame 100 has an outer surface 142 that is contoured to engage a native leaflet 914 of a heart valve 912, as described in detail below.

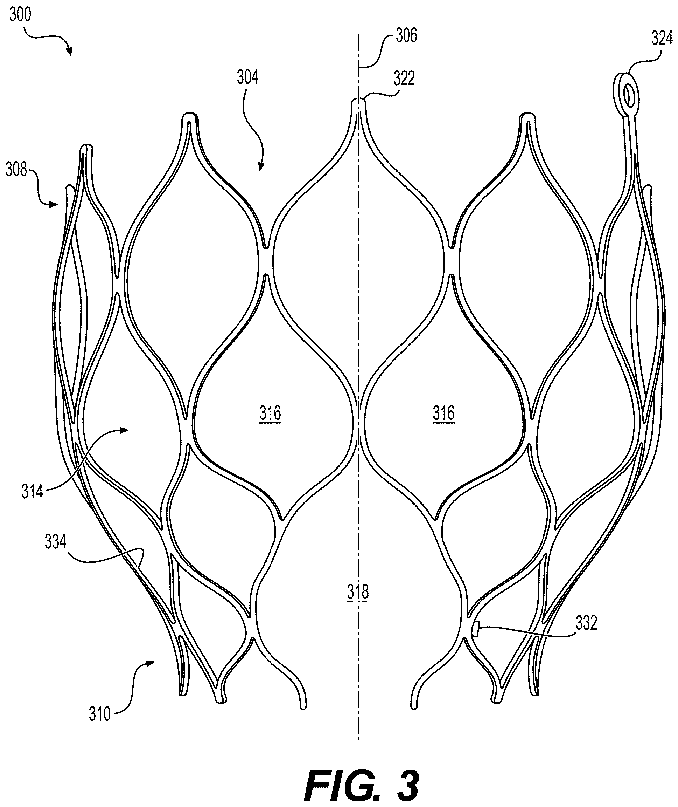

Referring to FIG. 3, an expandable anchoring frame 300 comprises one component of the transcatheter heart valve prosthesis 1400 (FIG. 14). The expandable anchoring frame 300 may be a self-expandable stent frame. In aspects, the expandable anchoring frame 300 can comprise a proximal end 308 (outflow end) and a distal end 310 (inflow end). The expandable anchoring frame 300 can extend from the proximal end 308 to the distal end 310. In aspects, the proximal end 308 can be configured to be received within the capsule 435 of the transcatheter delivery assembly 430 (see, FIG. 4). In some non-limiting examples, the proximal end 308 can be slidably disposed within the capsule 435. In some embodiments, the expandable anchoring frame 300 can be configured to achieve a contracted orientation (shown in FIG. 5) and a fully expanded orientation (shown in FIG. 3). In aspects, the expandable anchoring frame 300 can comprise an inner lumen 304 extending along an elongated axis 306.

The term "fully expanded orientation" should be construed to mean that the expandable anchoring frame 300 cannot expand any further with respect to the location at which the expandable anchoring frame 300 has been deployed (i.e., fully released from the transcatheter delivery assembly 430). In some cases, the term "fully expanded orientation" may refer to the expandable anchoring frame 300 not being capable of any further radial expansion regardless of the location at which the expandable anchoring frame 300 is deployed (e.g., the expandable anchoring frame 300 cannot radially expand any further, even when free from surrounding structure).

In some aspects, the expandable anchoring frame 300 can comprise a first plurality of cell structures 314. It should be understood that the expandable anchoring frame 300 is merely exemplary and any other suitable expandable stent frame may be utilized. Furthermore, the plurality of cell structures 314 should not be limited to cell structures that are each in the shape of a rhombus and should not be limited to the number of cell structures and/or size of the cell structures shown in FIG. 3. Accordingly, more or less suitable cell structures with a variety of different shapes and sizes can be employed.

In the example embodiment shown, the plurality of cell structures 314 include a plurality of cells 316 and at least one access cell 318. In particular, the at least one access cell 318 is larger than the cells 316 and can provide access to one or more coronary arteries when the transcatheter heart valve prosthesis 1400 is implanted in the patient. In the embodiment shown in FIG. 3, there is exactly one access cell 318. However, this is not meant to be limiting, as the expandable anchoring frame 300 of the transcatheter heart valve prosthesis 1400 can include more, fewer, or no access cells 318. For example, there may be two access cells 318, one configured to be aligned with each coronary artery. In another example, there may be three access cells 318, two of which are aligned with a coronary artery and a third in the non-coronary cusp. If there are three access cells 318, they may be evenly circumferentially spaced, in one embodiment. Further, the at least one access cell 318 may be located in other locations than the location shown in FIG. 3.

In some embodiments, as illustrated in FIG. 3, the expandable anchoring frame 300 can taper from the proximal end 308 of the expandable anchoring frame 300 to the distal end 310 of the expandable anchoring frame 300 such that a radial distance from an axis of the expandable anchoring frame 300 to an outer surface of the expandable anchoring frame 300 decreases from the proximal end 308 of the expandable anchoring frame 300 to the distal end 310 of the expandable anchoring frame 300. This is not meant to be limiting, however, as the expandable anchoring frame 300 can represent other shapes. Further, the tapering may not be continuous or constant, as long as the proximal end 308 has a larger radial distance than the distal end 310. The expandable anchoring frame 300, as illustrated in FIG. 3, can further includes a plurality of crowns 322 at the proximal end 308 of the expandable anchoring frame 300. In further aspects, at least one crown of the plurality of crowns 322 can comprise a hook or paddle 324 configured to release the expandable anchoring frame 300 from the transcatheter delivery assembly 430 (FIG. 4). The hook or paddle 324 of the expandable anchoring frame 300 will be discussed further below as the exemplary transcatheter delivery assembly 430 (FIG. 4) used with the transcatheter heart valve prosthesis 1400 (FIG. 14) is described. The expandable anchoring frame 300 comprises an inner surface 334 that is contoured to engage the native leaflets 914 of the heart valve 912, as described in detail below.

In further aspects, the expandable anchoring frame 300 can comprise one or more radiopaque markers 332 (e.g., one radiopaque marker, two radiopaque markers, three radiopaque markers, etc.), schematically illustrated in FIG. 3. In this way the expandable anchoring frame 300 can be located by a clinician (e.g., a surgeon) when maneuvering the expandable anchoring frame 300 through the vasculature and/or within a heart valve of a patient.

FIGS. 4 and 5 show schematically side views of a transcatheter delivery assembly 430 (e.g., "delivery assembly") for delivering and deploying a transcatheter heart valve prosthesis (e.g., the transcatheter heart valve prosthesis 1400) according to embodiments hereof. One skilled in the art will realize that FIGS. 4 and 5 illustrate one example of a transcatheter delivery assembly 430 and that components illustrated in FIGS. 4 and 5 may be removed and/or additional components may be added. The transcatheter delivery assembly 430 includes a distal end 431, a proximal end 432, and a handle 433. The handle 433 enables a physician to manipulate a distal portion of the transcatheter delivery assembly 430 and includes actuators for moving parts of the transcatheter delivery assembly 430 relative to other parts. In the transcatheter delivery assembly 430, an outer shaft 434 is coupled to an actuator 439 of the handle 433 for moving the outer shaft 434 relative to an inner shaft 536. A second actuator 442 on the proximal end 432 may be provided for allowing the inner shaft 536 and a distal tip or nose cone 437 to move distally and proximally relative to the handle 433 in a direction of arrow T. A third actuator 444 may be provided for allowing the clinician (e.g., the surgeon) to inflate a balloon 610 (FIG. 6) on the distal end 431.

A distal portion of the outer shaft 434, referred to as a capsule 435, is configured to surround a transcatheter heart valve prosthesis (e.g., the transcatheter heart valve prosthesis 1400) during delivery to the treatment site (e.g., a native heart valve) and is retracted from the transcatheter heart valve prosthesis to expose the transcatheter heart valve prosthesis such that it self-expands (in self-expanding embodiments). In this way, the capsule 435 is in frictional engagement with the transcatheter heart valve prosthesis 1400. In the embodiment shown, the inner shaft 536 includes a retainer 538 for receiving the paddles 324 of the expandable anchoring frame 300 of the transcatheter heart valve prosthesis 1400.

When the actuator 439 is actuated, the actuator 439 moves the outer shaft 434 and the capsule 435 relative to the inner shaft 536, as shown in FIG. 5. As known to those skilled in the art, when the transcatheter delivery assembly 430 is in position such that the transcatheter heart valve prosthesis 1400 is at the desired position at the treatment site in the patient's vasculature, the actuator 439 is actuated (e.g., rotated) to move the capsule 435 relative to the inner shaft 536 and the transcatheter heart valve prosthesis 1400 disposed between the inner shaft 536 and the capsule 435, thereby enabling the transcatheter heart valve prosthesis 1400 to deploy via self-expansion at the treatment site and release from the retainer 538, as shown in FIG. 5 (without showing the transcatheter heart valve prosthesis 1400).

In one embodiment, illustrated in FIG. 6, the expandable stent frame 100 and the expandable anchoring frame 300 are positioned in the capsule 435 of the transcatheter delivery assembly 430 with the expandable stent frame 100 being closer to the nose cone 437 than the expandable anchoring frame 300. The expandable stent frame 100 is collapsed around a balloon 610 and positioned outside of the expandable anchoring frame 300. In other words, the expandable stent frame 100 is axially spaced from the expandable anchoring frame 300. In this embodiment, when the capsule 435 is proximally withdrawn the expandable stent frame 100 is exposed and, as the capsule 435 continues to move proximally, the expandable anchoring frame 300 is exposed from the transcatheter delivery assembly 430. The capsule 435 is axially movable in a direction A, as illustrated in FIG. 6. A guidewire 604 can extend out of the nose cone 437 to aid the surgeon and directing the transcatheter delivery assembly 430 through the heart.

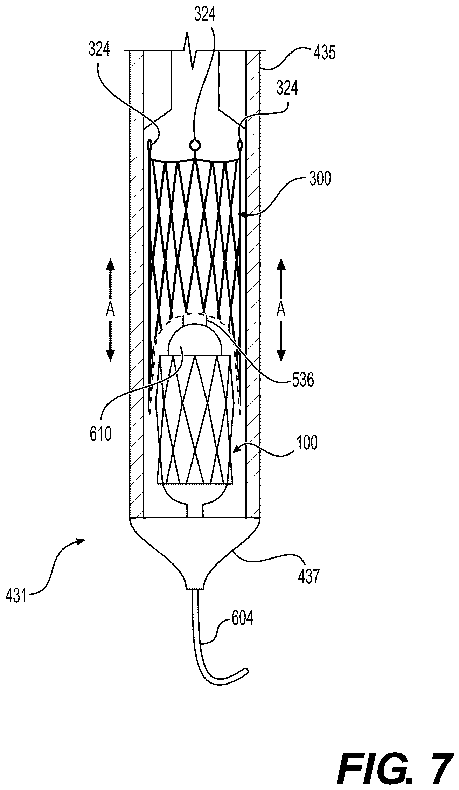

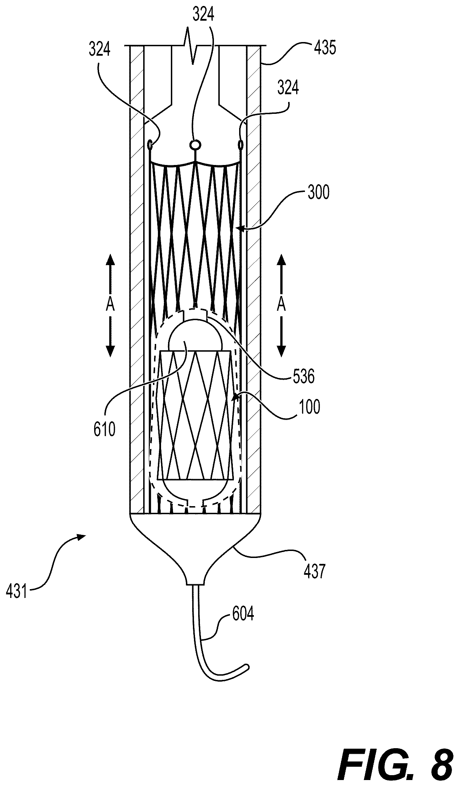

In another embodiment, illustrated in FIG. 7, the expandable stent frame 100 and the expandable anchoring frame 300 are positioned in the capsule 435 of the transcatheter delivery assembly 430 with the expandable stent frame 100 being partially placed inside of the expandable anchoring frame 300. The expandable stent frame 100 is collapsed around the balloon 610 and partially positioned inside of the expandable anchoring frame 300. In another embodiment, illustrated in FIG. 8, the expandable stent frame 100 and the expandable anchoring frame 300 are positioned in the capsule 435 of the transcatheter delivery assembly 430 with an entirely of the expandable stent frame 100 placed inside of the expandable anchoring frame 300. The expandable stent frame 100 is collapsed around the balloon 610 and completely positioned inside of the expandable anchoring frame 300. In each of the embodiments described above, the expansion of the expandable stent frame 100 may be controlled by the surgeon using the balloon 610 and the expansion of the expandable anchoring frame 300 may be controlled by the position of the capsule 435 relative to the paddle 324 of the expandable anchoring frame 300. The expandable anchoring frame 300 may not be expand until the capsule 435 is withdrawn to a position where the paddle 324 is free to disengage from the transcatheter delivery assembly 430. In each of the embodiments, the movement of the capsule 435 (as illustrated by the arrow A) may be used to deploy the expandable stent frame 100 and/or the expandable anchoring frame 300 from the transcatheter delivery assembly 430 and to recapture the expandable stent frame 100 and/or the expandable anchoring frame 300 into the transcatheter delivery assembly 430.

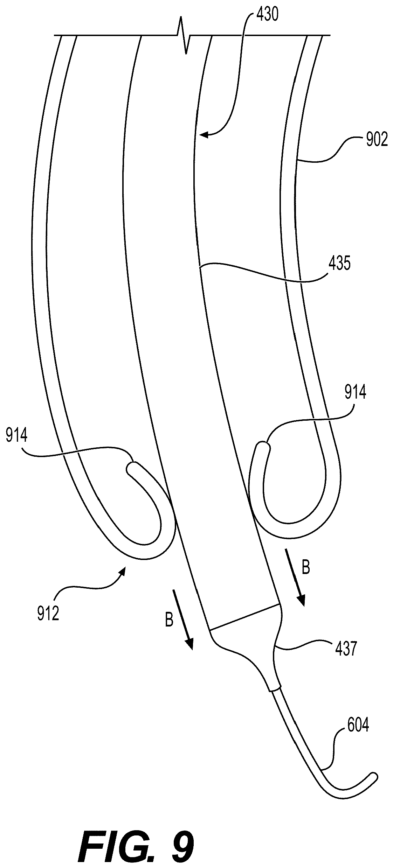

Methods of implanting the transcatheter heart valve prosthesis 1400 (FIG. 14) using the transcatheter delivery assembly 430 will now be described with reference to FIGS. 9-14. Methods will be discussed for implanting the transcatheter heart valve prosthesis 1400 (FIG. 14) with the understanding that, unless otherwise indicated, similar or identical methods can be employed for implanting the transcatheter heart valve prosthesis 1400 (FIG. 14) with any identifiable features previously mentioned regarding the expandable stent frame 100 and the expandable anchoring frame 300.

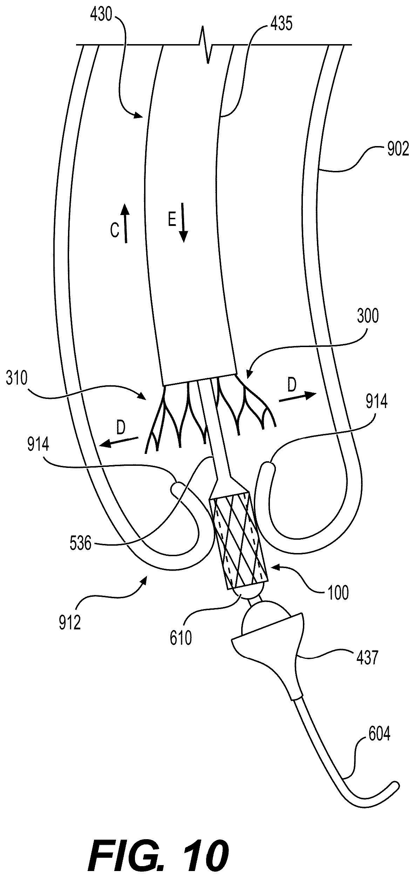

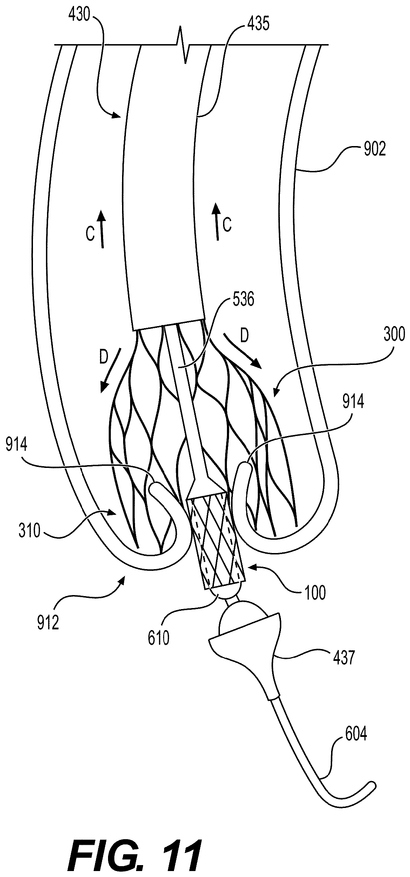

Referring to FIGS. 9-14, in one embodiment, the transcatheter heart valve prosthesis 1400 is positioned within a heart valve 912 by attaching the transcatheter heart valve prosthesis 1400 to the transcatheter delivery assembly 430 and inserting the transcatheter heart valve prosthesis 1400 past native leaflets 914 of the heart valve 912. The transcatheter heart valve prosthesis 1400 is delivered while the expandable stent frame 100 and the expandable anchoring frame 300 of the transcatheter heart valve prosthesis 1400 remain in the collapsed orientation on the inner shaft 536 of the transcatheter delivery assembly 430. As further illustrated in FIG. 9, the transcatheter delivery assembly 430 can be distally advanced in a direction of arrow B using the guidewire 604. Referring to FIGS. 10 and 11, the capsule 435 of the transcatheter delivery assembly 430 can be proximally withdrawn (in the direction of arrow C) such that the expandable stent frame 100 remains mounted on the inner shaft 536 in a contracted orientation while the distal end 310 of the expandable anchoring frame 300 can at least partially expand (in the direction D) above the native leaflets 914 of the heart valve 912. Once the distal end 310 has partially expanded in the direction D, the entire transcatheter delivery assembly 430 is advanced distally in the direction E until the distal end 310 of the expandable anchoring frame 300 seats behind the native leaflets 914, as illustrated in FIG. 11. In aspect, the second actuator 442 (FIG. 4) may be used to maintain the expandable stent frame 100 between the native leaflets 914 as the transcatheter delivery assembly 430 is distally advanced. In aspect, as the transcatheter delivery assembly 430 is moved distally, the second actuator 442 may be used to move the expandable stent frame 100 proximally (relative to the handle 433) so the expandable stent frame 100 can maintain its position between the native leaflets 914.

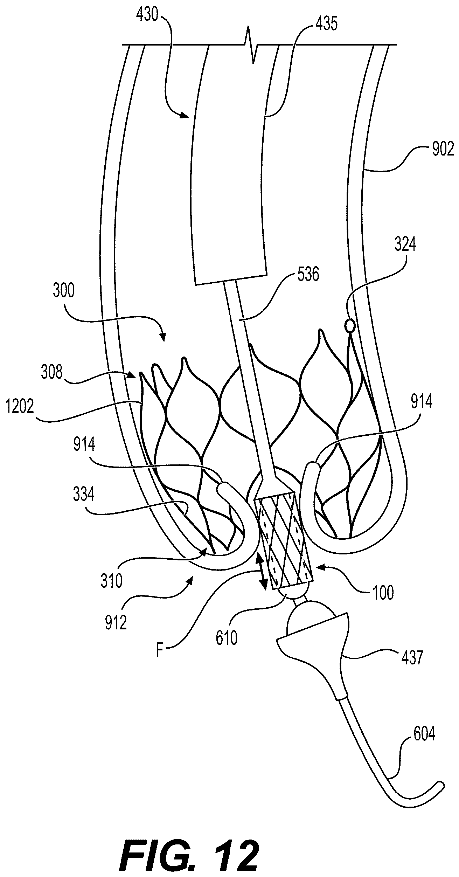

As further illustrated in FIG. 12, the capsule 435 can be further proximally withdrawn such that the expandable anchoring frame 300 is fully deployed from the capsule 435. Once the capsule 435 is proximally withdrawn to a proximal position coincident with paddle 324 of the expandable anchoring frame 300, the expandable anchoring frame 300 is automatically released from the transcatheter delivery assembly 430. Once the expandable anchoring frame 300 is released, the expandable anchoring frame 300 can expand such that an exterior surface 1202 of the expandable anchoring frame 300 contacts an adjacent anatomy 902 (FIG. 9) of the heart. As depicted in FIG. 12, the expandable anchoring frame 300 can be contoured, e.g., tapered from the proximal end 308 of the expandable anchoring frame 300 to the distal end 310 of the expandable anchoring frame 300, as described in detail above. Further, prior to full release of the expandable anchoring frame 300, the capsule 435 can be distally advanced to recapture the expandable anchoring frame 300 back into the capsule 435 for realignment of the expandable anchoring frame 300, if needed. To align the expandable anchoring frame 300, the radiopaque markers 332 (FIG. 3) can be used to help properly align the at least one access cell 318 of the expandable anchoring frame 300 with coronary arteries. The at least one access cell 318 of the expandable anchoring frame 300 is larger than the cells 316, as described in detail above, in order to provide better access for blood flow from coronary arteries.

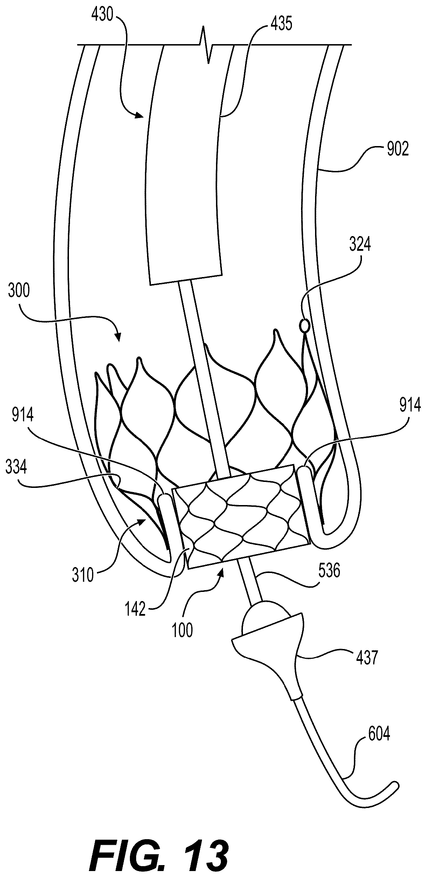

Referring to FIG. 12, once the expandable anchoring frame 300 has been properly aligned and fully released, the expandable stent frame 100 can be further aligned with a plurality of native leaflets 914 the heart valve 912, if needed, using the second actuator 442. The second actuator 442 may be moved distally and proximally (relative to the handle 433) in the direction of arrow T (FIG. 4) to move the inner shaft 536 and the expandable stent frame 100 distally and proximally in the direction of arrow F. Referring to FIG. 13, in some embodiments, the expandable stent frame 100 is balloon-expandable. In embodiments where the expandable stent frame 100 is balloon-expandable, the balloon 610, positioned within the expandable stent frame 100 can be inflated by introducing fluid through a fluid conduit at a location exterior of the patient. For example, the transcatheter delivery assembly 430 can comprise a fluid port 446 on the proximal end 432 that is in fluid communication with an interior of the balloon 610 positioned within the interior of the expandable stent frame 100. In some embodiments, the third actuator 444 can open a valve (not shown) to introduce pressurized fluid from the conduit in fluid communication with a source of pressurized fluid to inflate the expandable stent frame 100. For example, the third actuator 444 can be engaged by a surgeon to open a valve to allow pressurized fluid to flow from the source of pressurized fluid to the inner shaft 536 and inflate the balloon 610 to a predetermined shape. In other embodiments, the expandable stent frame 100 can be self-expanding and comprise nitinol.

As further shown in FIG. 13, the expandable stent frame 100 is positioned such that upon expanding, the expandable stent frame 100 can capture at least one native leaflet 914 of the heart valve 912 between the expandable stent frame 100 and the expandable anchoring frame 300. When the expandable stent frame 100 is fully expanded, the at least one native leaflet 914 may be constrained between the inner surface 334 of the expandable anchoring frame 300 and the outer surface 142 of the expandable stent frame 100. FIG. 14 further illustrates removal of the transcatheter delivery assembly 430 (in the direction of arrow G) upon full delivery of the transcatheter heart valve prosthesis 1400 capturing at least one native leaflet 914 of the heart valve 912 between the expandable anchoring frame 300 and the expandable stent frame 100.

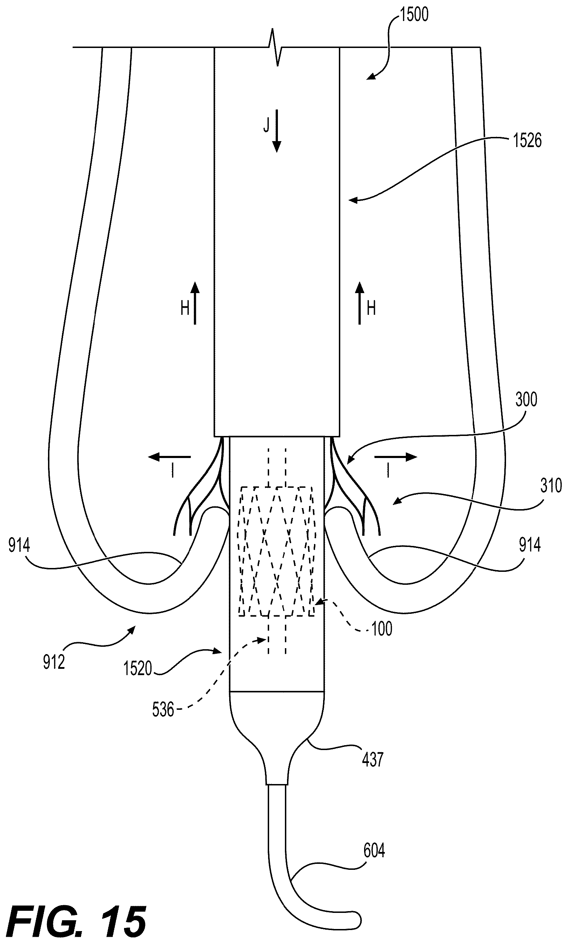

Referring now to FIGS. 15-19, in another embodiment a transcatheter delivery assembly 1500 includes an inner sheath 1520. The inner sheath 1520 may further be positioned along a length of the inner shaft 536 and within an outer sheath 1526. The outer sheath 1526 may circumferentially enclose the expandable anchoring frame 300 of the transcatheter heart valve prosthesis 1400 in a contracted orientation prior to deployment. The expandable anchoring frame 300 is then positioned around the inner sheath 1520 and within the outer sheath 1526 in the contracted orientation prior to deployment. The inner sheath 1520 may circumferentially enclose the expandable stent frame 100 of the transcatheter heart valve prosthesis 1400. The expandable stent frame 100 may be mounted along the inner shaft 536 of the transcatheter delivery assembly 1500. In aspects, the expandable stent frame 100 is self-expanding. In some embodiments of the transcatheter delivery assembly 1500, the expandable stent frame 100 is balloon-expandable.

FIGS. 15-19 further illustrate a method of deploying the transcatheter heart valve prosthesis 1400 wherein the transcatheter delivery assembly 1500 includes the inner sheath 1520. Referring to FIG. 15 the transcatheter delivery assembly 1500 is distally advanced using the guidewire 604 to place the inner sheath 1520 between the native leaflets 914 of the heart valve 912. Once the transcatheter delivery assembly 1500 is positioned, the outer sheath 1526 of the transcatheter delivery assembly 1500 can be proximally withdrawn (in the direction of arrow H) such that the expandable stent frame 100 remains mounted on the inner shaft 536 in the contracted orientation inside of the inner sheath 1520 while the distal end 310 of the expandable anchoring frame 300 can be at least partially expanded above the native leaflets 914 of the heart valve 912 (see arrow I).The entire transcatheter delivery assembly 1500 is then distally advanced in the direction of arrow J to seat the distal end 310 of the expandable anchoring frame 300 behind the native leaflets 914 of the heart valve 912.

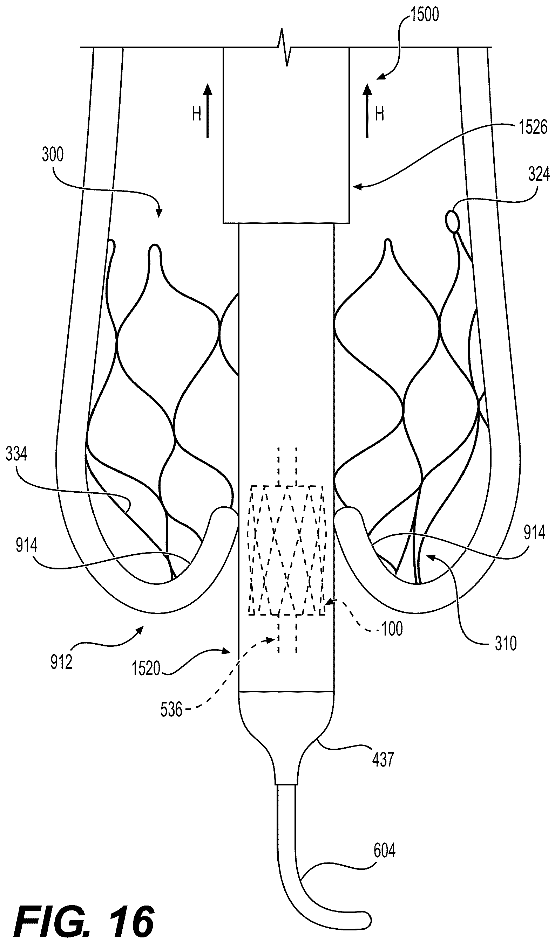

As further illustrated in FIG. 16, once the expandable anchoring frame 300 is properly positioned with the distal end 310 behind the native leaflets 914, the outer sheath 1526 can be further proximally withdrawn such that the expandable anchoring frame 300 is fully deployed from the outer sheath 1526 adjacent the heart valve 912. Once the outer sheath 1526 is proximally withdrawn to a proximal position coincident with releasing the paddle 324 of the expandable anchoring frame 300 from the transcatheter delivery assembly 1500, the expandable anchoring frame 300 expands such that the inner surface 334 of the expandable anchoring frame 300 is positioned adjacent the at least one native leaflet 914 of the heart valve 912. Further, prior to full release of the expandable anchoring frame 300, the outer sheath 1526 may be distally advanced to recapture the expandable anchoring frame 300 back into the outer sheath 1526 for realignment of the expandable anchoring frame 300, if needed. To align the expandable anchoring frame 300, the expandable anchoring frame 300 can further include at least one radiopaque marker 332 (FIG. 3) to help properly align the at least one access cell 318 (FIG. 3) of the expandable anchoring frame 300 with the coronary artery of the patient.

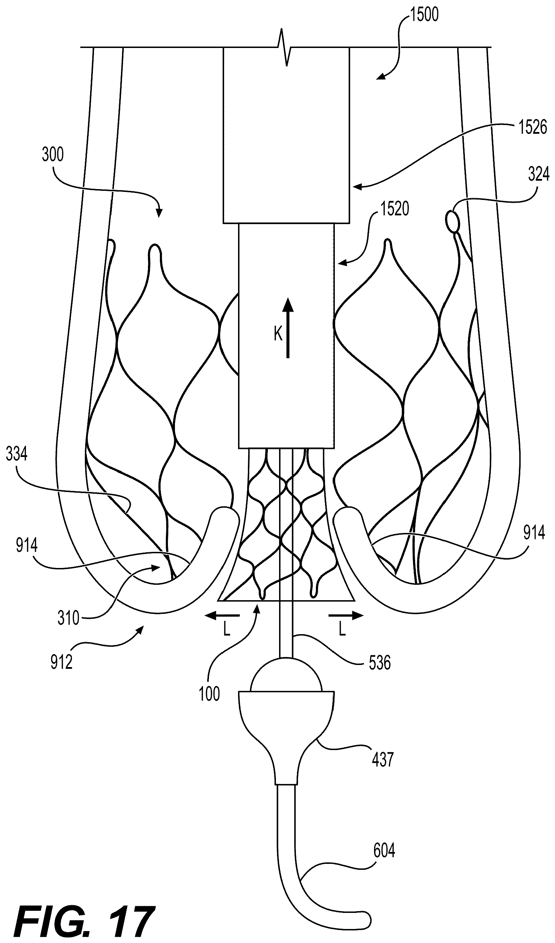

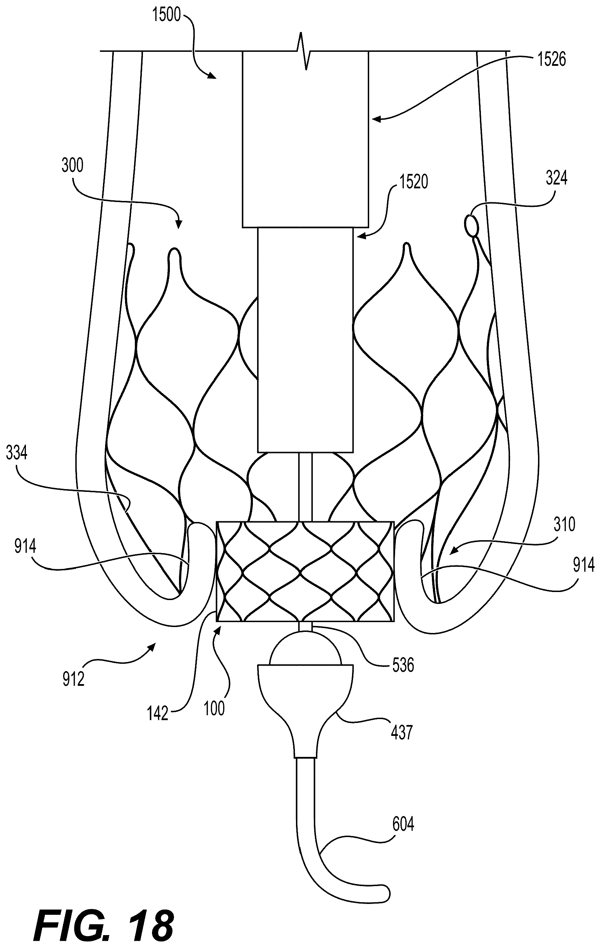

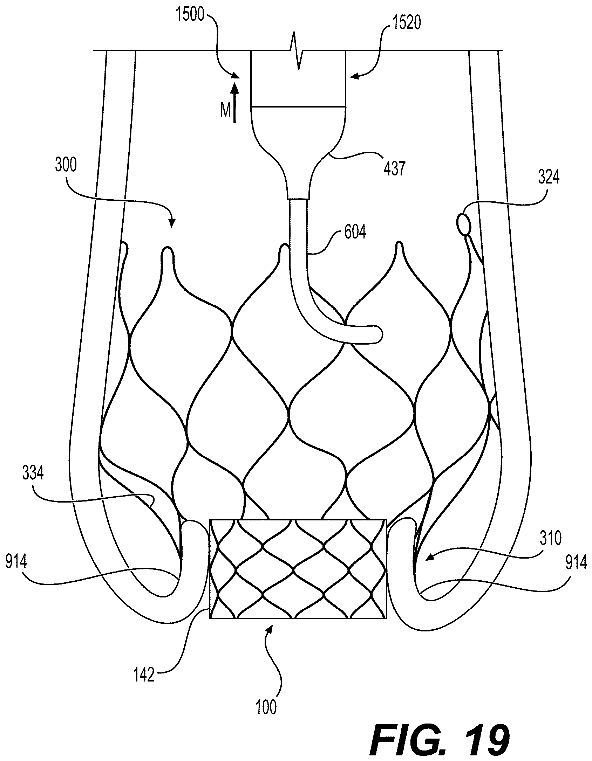

Referring to FIG. 17, the inner sheath 1520 is proximally withdrawn (in the direction of arrow K) such that at least part of the expandable stent frame 100 is released within the heart valve 912 (in the direction of arrows L). In some embodiments, the expandable stent frame 100 is balloon-expandable. The expandable stent frame 100 expands to a predetermined shape and size as shown in FIG. 18. Once fully expanded, the expandable stent frame 100 is positioned such that the outer surface 142 of the expandable stent frame 100 can move the at least one native leaflet 914 of the heart valve 912 and capture the at least one native leaflet 914 between the outer surface 142 of the expandable stent frame 100 and the inner surface 334 of the expandable anchoring frame 300. FIG. 19 further illustrates removal of the transcatheter delivery assembly 1500 in the direction of arrow M upon full delivery of the transcatheter heart valve prosthesis 1400 pinning the at least one native leaflet 914 of the heart valve 912 between the expandable anchoring frame 300 and the expandable stent frame 100.

Example 1: A heart valve prosthesis for capturing at least one native leaflet of a heart valve. The heart valve prosthesis comprises an expandable anchoring frame comprising an interior area extending between a proximal portion of the expandable anchoring frame and a distal portion of the expandable anchoring frame. An expandable stent frame comprises an interior area extending between a proximal portion of the expandable stent frame and a distal portion of the expandable stent frame. The expandable stent frame comprises a prosthetic valve structure mounted within the interior area of the expandable stent frame. The expandable stent frame is configured to be inserted into the interior area of the expandable anchoring frame and, when the expandable stent frame is in an expanded configuration, the expandable stent frame is biased radially outward toward an inner surface of the expandable anchoring frame and is dimensioned to capture the at least one native leaflet between an outer surface of the expandable stent frame and the inner surface of the expandable anchoring frame.

Example 2: The heart valve prosthesis of Example 1, wherein the expandable anchoring frame tapers from the proximal portion of the expandable anchoring frame to the distal portion of the expandable anchoring frame such that a diameter of the proximal portion of the expandable anchoring frame is less than a diameter of the distal portion of the expandable anchoring frame .

Example 3: The heart valve prosthesis of any one of Examples 1-2, wherein the expandable anchoring frame includes a network of struts defining a plurality of open cells, and wherein at least one open cell of the plurality of open cells defines a coronary access port.

Example 4: The heart valve prosthesis of any one of Examples 1-3, wherein the proximal portion of the expandable anchoring frame includes at least one paddle for releasably securing the expandable anchoring frame to a transcatheter delivery assembly.

Example 5: The heart valve prosthesis of any one of Examples 1-4, wherein the expandable anchoring frame is self-expanding.

Example 6: The heart valve prosthesis of Example 5, wherein the expandable anchoring frame comprises nitinol.

Example 7: The heart valve prosthesis of any one of Examples 1-6, wherein the expandable anchoring frame further includes radiopaque markers.

Example 8: The heart valve prosthesis of any one of Examples 1-7, wherein the expandable stent frame is balloon expandable.

Example 9: The heart valve prosthesis of any one of Examples 1-7, wherein the expandable stent frame is self-expanding.

Example 10: A method of implanting the heart valve prosthesis of Example 1 comprising: delivering the heart valve prosthesis to the heart valve of a patient while both the expandable anchoring frame and the expandable stent frame are in contracted orientations; fully deploying the expandable anchoring frame such that at least one leaflet of the heart valve is adjacent the inner surface of the expandable anchoring frame; expanding the expandable anchoring frame; fully deploying the expandable stent frame into the interior area of the expandable anchoring frame such that the at least one leaflet of the heart valve is positioned between the inner surface of the expandable anchoring frame and the outer surface of the expandable stent frame; and expanding the expandable stent frame to capture the at least one leaflet of the heart valve between the expandable anchoring frame and the expandable stent frame.

Example 11: The method of Example 10, wherein the step of delivering the heart valve prosthesis to the heart valve of a patient includes positioning the heart valve prosthesis in a transcatheter delivery assembly.

Example 12: The method of Example 10, wherein the step of fully deploying the expandable anchoring frame includes aligning coronary access ports of the expandable anchoring frame with coronary arteries of a heart.

Example 13: The method of any one of Examples 10-12, wherein the step of fully deploying the expandable anchoring frame includes aligning at least one radiopaque marker of the expandable anchoring frame with a predetermined location in a heart.

Example 14: The method of any one of Examples 10-13, wherein the step of fully deploying the expandable anchoring frame includes retracting a sheath from around an outer surface of the expandable anchoring frame.

Example 15: The method of any one of Examples 10-13, wherein the step of fully deploying the expandable anchoring frame includes: partially retracting a sheath from around an outer surface of the expandable anchoring frame; adjusting a position of the expandable anchoring frame; and fully retracting the sheath from around the outer surface of the expandable anchoring frame.

Example 16: The method of any one of Examples 10-15, wherein the step of fully deploying the expandable anchoring frame includes disconnecting paddles of the expandable anchoring frame from a transcatheter delivery assembly.

Example 17: The method of any one of Examples 10-16, wherein the expandable stent frame is positioned within an inner sheath when in the contracted orientation.

Example 18: The method of Example 17, wherein the step of fully deploying the expandable stent frame includes retracting the inner sheath from around the expandable stent frame.

Example 19: The method of any one of Examples 10-18, wherein the expandable anchoring frame is self-expanding.

Example 20: The method of any one of Examples 10-19, wherein the expandable anchoring frame comprises nitinol.

Example 21: The method of any one of Examples 10-20, wherein the expandable anchoring frame further includes radiopaque markers.

Example 22: The method of any one of Examples 10-21, wherein the expandable stent frame is balloon expandable.

Example 23: The method of any one of Examples 10-21, wherein the expandable stent frame is self-expanding.

Example 24: A two-component prosthetic valve delivery assembly comprising: a transcatheter delivery assembly comprising: an outer sheath, and an inner member, wherein the outer sheath and the inner member are movable relative to each other in an axial direction. An expandable anchoring frame is constrained by the outer sheath. An expandable stent frame comprises a prosthetic valve structure mounted within an interior area of the expandable stent frame. The expandable stent frame is disposed between the outer sheath and the inner member and moveable with the inner member. The expandable anchoring frame is configured to be released from the transcatheter delivery assembly prior to the expandable stent frame being released from the transcatheter delivery assembly.

Example 25: The two-component prosthetic valve delivery assembly of Example 24, wherein the expandable stent frame is balloon expandable.

Example 26: The two-component prosthetic valve delivery assembly of any one of Examples 24-25, wherein expandable stent frame is disposed distal to the expandable anchoring frame in a pre-delivery configuration.

Example 27: The two-component prosthetic valve delivery assembly of Example 24, wherein the expandable stent frame is self-expanding and a second sheath is disposed around the expandable stent frame to constrain the expandable stent frame.

It should be understood that while various aspects have been described in detail relative to certain illustrative and specific examples thereof, the present disclosure should not be considered limited to such, as numerous modifications and combinations of the disclosed features are possible without departing from the scope of the following claims.

## Claims

1. A heart valve prosthesis (1400) for capturing at least one native leaflet (914) of a heart valve (912), the heart valve prosthesis (1400) comprising:
an expandable anchoring frame (300) comprising an interior area extending between a proximal portion (308) of the expandable anchoring frame (300) and a distal portion (310) of the expandable anchoring frame (300); and
an expandable stent frame (100) comprising an interior area extending between a proximal portion (108) of the expandable stent frame (100) and a distal portion (110) of the expandable stent frame (100), the expandable stent frame (100) comprising a prosthetic valve structure (220) mounted within the interior area of the expandable stent frame (100),
wherein the expandable stent frame (100) is configured to be inserted into the interior area of the expandable anchoring frame (300) and, when the expandable stent frame (100) is in an expanded configuration, the expandable stent frame (100) is biased radially outward toward an inner surface (334) of the expandable anchoring frame (300) and is dimensioned to capture the at least one native leaflet (914) between an outer surface (142) of the expandable stent frame (100) and the inner surface (334) of the expandable anchoring frame (300).

2. The heart valve prosthesis (1400) of claim 1, wherein the expandable anchoring frame (300) tapers from the proximal portion (308) of the expandable anchoring frame(300) to the distal portion (310) of the expandable anchoring frame (300) such that a diameter of the proximal portion (308) of the expandable anchoring frame (300) is greater than a diameter of the distal portion (310) of the expandable anchoring frame (300).

3. The heart valve prosthesis (1400) of any one of claims 1-2, wherein the expandable anchoring frame (300) includes a network of struts defining a plurality of open cells (316), and wherein at least one open cell (318) of the plurality of open cells (316) defines a coronary access port.

4. The heart valve prosthesis (1400) of any one of claims 1-3, wherein the expandable anchoring frame (300) is self-expanding.

5. The heart valve prosthesis (1400) of any one of claims 1-4, wherein the expandable stent frame (100) is balloon expandable.

6. The heart valve prosthesis (1400) of any one of claims 1-4, wherein the expandable stent frame (100) is self-expanding.

7. A method of implanting the heart valve prosthesis (1400) of claim 1, comprising:
delivering the heart valve prosthesis (1400) to the heart valve (912) of a patient while both the expandable anchoring frame (300) and the expandable stent frame (100) are in contracted orientations;
fully deploying the expandable anchoring frame (300) such that at least one leaflet (914) of the heart valve (912) is adjacent the inner surface (334) of the expandable anchoring frame (300);
expanding the expandable anchoring frame (300);
fully deploying the expandable stent frame (100) into the interior area of the expandable anchoring frame (300) such that the at least one leaflet (914) of the heart valve (912) is positioned between the inner surface (334) of the expandable anchoring frame (300) and the outer surface (142) of the expandable stent frame (100); and
expanding the expandable stent frame (100) to capture the at least one leaflet (914) of the heart valve (912) between the expandable anchoring frame (300) and the expandable stent frame (100).

8. The method of claim 7, wherein the step of delivering the heart valve prosthesis (1400) to the heart valve (912) of a patient includes positioning the heart valve prosthesis (1400) in a transcatheter delivery assembly (430, 1500).

9. The method of claim 7, wherein the step of fully deploying the expandable anchoring frame (300) includes aligning coronary access ports of the expandable anchoring frame (300) with coronary arteries of a heart.

10. The method of any one of claims 7-9, wherein the step of fully deploying the expandable anchoring frame (300) includes aligning at least one radiopaque marker (332) of the expandable anchoring frame (300) with a predetermined location in a heart.

11. The method of any one of claims 7-10, wherein the step of fully deploying the expandable anchoring frame (300) includes retracting a sheath (435, 1526) from around an outer surface of the expandable anchoring frame (300).

12. The method of any one of claims 7-10, wherein the step of fully deploying the expandable anchoring frame (300) includes:
partially retracting a sheath (435, 1526) from around an outer surface of the expandable anchoring frame (300);
adjusting a position of the expandable anchoring frame (300); and
fully retracting the sheath (435, 1526) from around the outer surface of the expandable anchoring frame (300).

13. The method of any one of claims 7-12, wherein the step of fully deploying the expandable anchoring frame (300) includes disconnecting paddles (324) of the expandable anchoring frame (300) from a transcatheter delivery assembly (430, 1500).

14. The method of any one of claims 7-13, wherein the expandable stent frame (100) is positioned within an inner sheath (1520) when in the contracted orientation.

15. The method of claim 14, wherein the step of fully deploying the expandable stent frame (100) includes retracting the inner sheath (1520) from around the expandable stent frame (100).
